# EUROPEAN PATENT SPECIFICATION

(11) **EP 1 799 117 B1**
(45) Date of publication and mention of the grant of the patent: **04.01.2017**
(21) Application number: 05787214.5
(22) Date of filing: 03.10.2005
(51) Int. Cl.: A61B 8/14, G01S 15/89, G01S 7/52

(54) **THREE DIMENSIONAL DIAGNOSTIC ULTRASOUND IMAGING SYSTEM WITH IMAGE REVERSAL AND INVERSION**
DREIDIMENSIONALES DIAGNOSTISCHES ULTRASCHALLDARSTELLUNGSSYSTEM MIT BILDUMKEHR UND INVERSION
SYSTÈME D'IMAGERIE DIAGNOSTIQUE ULTRASONORE EN TROIS DIMENSIONS AVEC RENVERSEMENT ET INVERSION D'IMAGE

(30) Priority: 08.10.2004 US 617492 P
(43) Date of publication of application: 27.06.2007
(73) Proprietor: Koninklijke Philips N.V., 5656 AE Eindhoven (NL)
(72) Inventor: FRISA, Janice, Bothell, Washington 98041-3003 (US); THIELE, Karl, Bothell, Washington 98041-3003 (US); PRATER, David, Bothell, Washington 98041-3003 (US); LIU, Larry Lingnan, Bothell, Washington 98041-3003 (US)
(74) Representative: Roche, Denis
(86) International application number: PCT/IB2005/053249
(87) International publication number: WO 2006/038180

(56) References cited:
- EP-A- 1 419 737
- WO-A-03/045222
- WO-A-2004/029655
- US-A1- 2003 156 746
- US-B1- 6 245 017
- PATENT ABSTRACTS OF JAPAN vol. 2000, no. 13, 5 February 2001 (2001-02-05) -& JP 2000 287976 A (OLYMPUS OPTICAL CO LTD), 17 October 2000 (2000-10-17)

## Description

This invention relates to ultrasonic diagnostic imaging and, in particular, to a three dimensional ultrasonic imaging system in which 3D images can be easily inverted and reversed for viewing from different diagnostic perspectives.

Live, real time 3D imaging has been commercially available for several years. Live 3D imaging, even more than standard 2D imaging, poses tradeoffs of image quality versus frame rate. For good image quality it is desirable to transmit and receive a large number of well-focused scan lines over the image field. For high real time frame rate, particularly useful when imaging a moving object such as the heart, it is desirable to transmit and receive all of the scan lines for an image in a short period of time. However, the transmission and reception of scan lines is limited by the laws of physics governing the speed of sound to 1540 m/sec. Thus, depending upon the depth of the image (which determines the time needed to wait for the return of the echoes over the full depth of the image), a determinable amount of time is required to transmit and receive all of the scan lines for an image, which may cause the frame rate of display to be unacceptably low. A solution to this problem is to reduce the number of scan lines and increase the degree of multiline reception. This will increase the frame rate, but possibly at the expense of degradation in the image quality. In 3D imaging the problem is even more acute, as hundreds or thousands of scan lines may be needed to fully scan a volumetric region. Another solution which reduces the number of scan lines is to narrow the volume being scanned, which also increases the frame rate. But this may undesirably provide only a view of a small section of the anatomy which is the subject of the ultrasonic exam.

As previously mentioned, this dilemma presents itself most starkly when imaging a moving object such as the beating heart. An ingenious solution to the dilemma for 3D imaging of the heart is described in US Pat. 5,993,390. The approach taken in this patent is to divide the cardiac cycle into twelve phases. A region of the heart which is scanned during one-twelfth of the cardiac cycle will produce a substantially stationary (unblurred) image. The inventors in the patent determined that nine such regions comprise the full volume of the typical heart. Thus, the heart is scanned to acquire one of these nine subvolumes during each of the twelve phases of the heart cycle. Over a period of nine heartbeats a complete 3D image of the heart is pieced together from the subvolumes for each of the twelve phases of the heart cycle. When the complete images are displayed in real time in phase succession, the viewer is presented with a real time image of the heart. This is a replayed image, however, and not a current live image of the heart. It would be desirable to enable current live 3D imaging of a volumetric region sufficient to encompass the heart.

WO 2004/029655 A describes three-dimensional ultrasound-imaging system and method that aim at providing an increased frame rate or an optimized image-acquisition time.

In accordance with the principles of the present disclosure, current live subvolumes of the heart are acquired in real time. The subvolumes can be steered over a maximum volumetric region while the ultrasound probe is held stationary at a chosen acoustic window. This enables the user to find the best acoustic region for viewing the maximum volumetric region, then to interrogate the region by steering live 3D subvolumes over it. In one embodiment the subvolumes are steerable over predetermined incremental positions. In another embodiment the subvolumes are continuously steerable over the maximum volumetric region. A first display embodiment is described with concurrent 3D and 2D images that enable the user to intuitively sense the location of the subvolume. Another display embodiment is described which enables the user to select from among a number of desirable viewing orientations.

In the drawings:
FIGURE 1 illustrates an ultrasonic diagnostic imaging system constructed in accordance with the principles of the present disclosure.
FIGURE 2 illustrates in block diagram form the architecture of an ultrasound system constructed in accordance with the principles of the present disclosure.
FIGURE 3 illustrates in block diagram form the major elements of a 3D probe and beamformer in one embodiment of the present disclosure.
FIGURE 4 illustrates a volumetric region which can be scanned from a two-dimensional matrix transducer.
FIGURE 5 illustrates a volumetric region encompassing the heart in an apical view.
FIGURE 6 illustrates the division of the volumetric region of FIGURES 4 and 5 into three subvolumes.
FIGURE 7 illustrates elevation planes of the subvolumes of FIGURE 6.
FIGURES 8a-8c are ultrasonic images of the three subvolumes of FIGURE 6.
FIGURES 9a-9c illustrate the beam plane inclination used to scan the three subvolumes of FIGURES 8a-8c.
FIGURE 10 illustrates the multiline reception used in the acquisition of the three subvolumes of FIGURES 8a-8c.
FIGURES 11-22 are screen shots of two and three dimensional images in different orientations in accordance with the present disclosure; and
FIGURES 11a-22a illustrate the views of the heart which may be obtained with the image orientations of FIGURES 11-22.
FIGURE 23 is a block diagram illustrating the control sequence for continuous steering of a subvolume over a maximum volumetric region.
FIGURE 24 illustrates a subvolume repositioned by continuous steering.

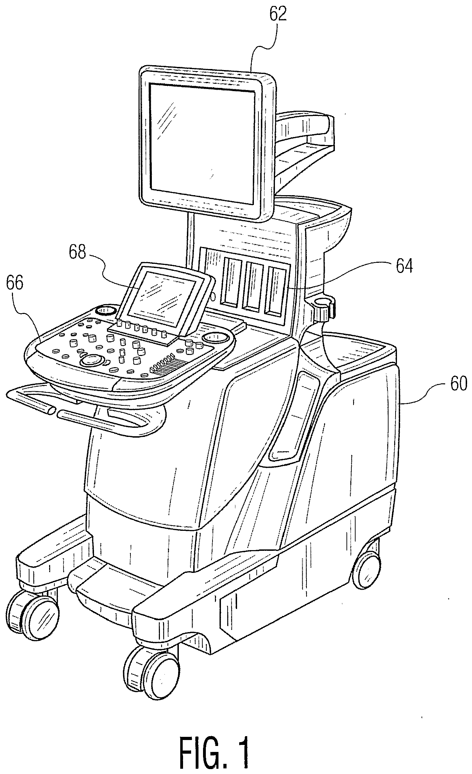

Referring first to FIGURE 1, an ultrasound system constructed in accordance with the principles of the present disclosure is shown. The ultrasound system includes a mainframe or chassis 60 containing most of the electronic circuitry for the system. The chassis 60 is wheel-mounted for portability. An image display 62 is mounted on the chassis 60. Different imaging probes may be plugged into three connectors 64 on the chassis. The chassis 60 includes a control panel with a keyboard and controls, generally indicated by reference numeral 66, by which a sonographer operates the ultrasound system and enters information about the patient or the type of examination that is being conducted. At the back of the control panel 66 is a touchscreen display 68 on which programmable softkeys are displayed for specific control function as described below. The sonographer selects a softkey on the touchscreen display 18 simply by touching the image of the softkey on the display. At the bottom of the touchscreen display is a row of buttons, the functionality of which varies in accordance with the softkey labels on the touchscreen immediately above each button.

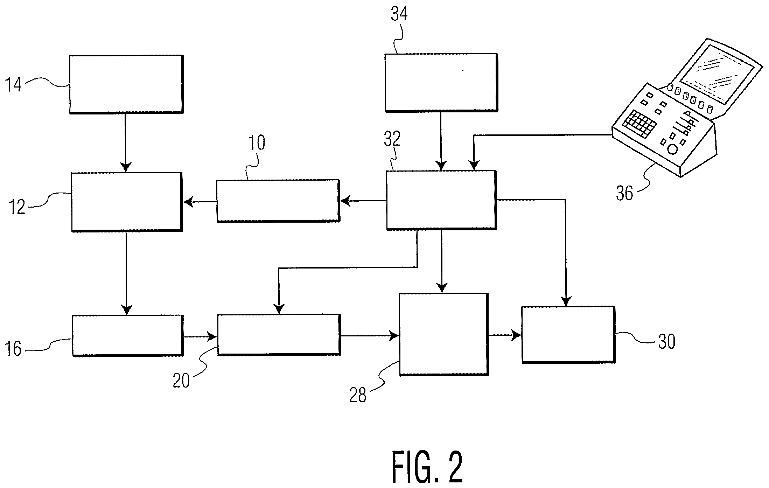

A block diagram of the major elements of an ultrasound system of the present disclosure is shown in FIGURE 2. An ultrasound transmitter 10 is coupled through a transmit/receive (T/R) switch 12 to a transducer array 14. Transducer array 14 is a two-dimensional array (matrix array) of transducer elements for performing three-dimensional scanning. The transducer array 14 transmits ultrasound energy into a volumetric region being imaged and receives reflected ultrasound energy, or echoes, from various structures and organs within the region. The transmitter 10 includes a transmit beamformer which controls the delay timing by which the signals applied to elements of the transducer array are timed to transmit beams of a desired steering direction and focus. By appropriately delaying the pulses applied to each transducer element by transmitter 10, the transmitter 10 transmits a focused ultrasound beam along a desired transmit scan line. The transducer array 14 is coupled through T/R switch 12 to an ultrasound receiver 16. Reflected ultrasound energy from points within the volumetric region is received by the transducer elements at different times. The transducer elements convert the received ultrasound energy to received electrical signals which are amplified by receiver 16 and supplied to a receive beamformer 20. The signals from each transducer element are individually delayed and then are summed by the beamformer 20 to provide a beamformed signal that is a representation of the reflected ultrasound energy level along points on a given receive scan line. As known in the art, the delays applied to the received signals may be varied during reception of ultrasound energy to effect dynamic focusing. The process is repeated for multiple scan lines to directed throughout the volumetric region to provide signals for generating an image of the volumetric region. Because the transducer array is two-dimensional, the receive scan lines can be steered in azimuth and in elevation to form a three-dimensional scan pattern. The beamformed signals may undergo signal processing such as filtering and Doppler processing and are stored in an image data buffer 28 which stores image data for different volume segments or subvolumes of a maximum volumetric region. The image data is output from image data buffer 28 to a display system 30 which generates a three-dimensional image of the region of interest from the image data for display on the image display 62. The display system 30 includes a scan converter which converts sector scan signals from beamformer 20 to conventional raster scan display signals. The display system 30 also includes a volume renderer. A system controller 32 provides overall control of the system in response to user inputs and internally stored data. The system controller 32 performs timing and control functions and typically includes a microprocessor and associated memory. The system controller is also responsive to signals received from the control panel and touchscreen display 36 through manual or voice control by the system user. An ECG device 34 includes ECG electrodes attached to the patient. The ECG device 34 supplies ECG waveforms to system controller 32 for display during a cardiac exam. The ECT signals may also be used during certain exams to synchronize imaging to the patient's cardiac cycle.

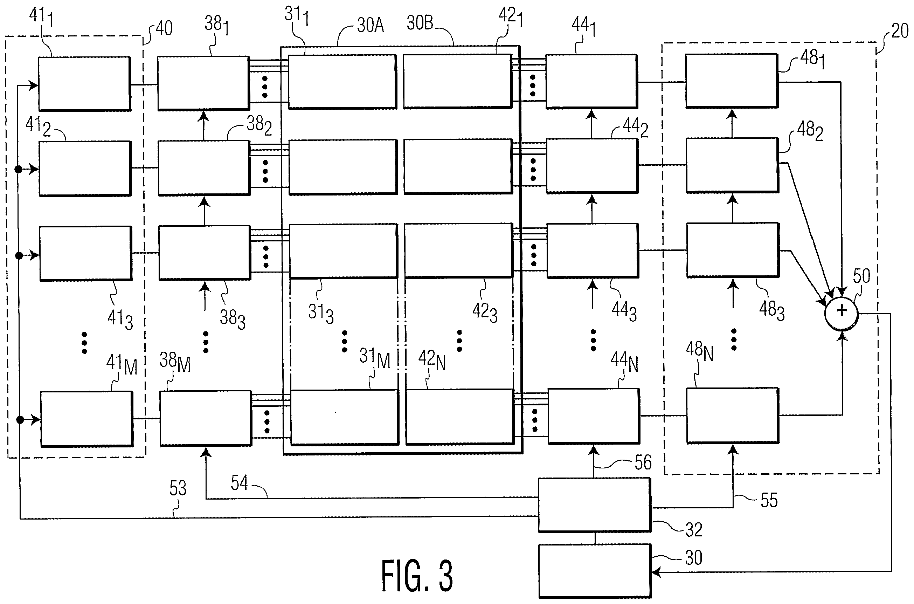

FIGURE 3 is a more detailed block diagram of an ultrasound system when operating with a matrix array for 3D imaging. The elements of the two-dimensional transducer array 14 of FIGURE 1 are divided into M transmit sub-arrays 30A connected to M intra-group transmit processors and N receive sub-arrays 30B connected to N intra-group receive processors. Specifically, transmit sub-arrays 31₁, 31₂, ... , 31_{M} are connected to intra-group transmit processors 38₁, 38₂, ... , 38_{M}, respectively, which in turn are connected to channels 41₁, 41₂, ... , 41_{M} of a transmit beamformer 40. Receive sub-arrays 42₁, 42₂, ... , 42_{N} are connected to intra-group receive processors 44₁, 44₂, ... , 44_{N}, respectively, which, in turn, are connected to processing channels 48₁, 48₂, ... , 48_{N} of a receive beamformer 20. Each intra-group transmit processor 38ᵢ includes one or more digital waveform generators that provide the transmit waveforms and one or more voltage drivers that amplify the transmit pulses to excite the connected transducer elements. Alternatively, each intra-group transmit processor 38ᵢ includes a programmable delay line receiving a signal from a conventional transmit beamformer. For example, transmit outputs from the transmitter 10 may be connected to the intra-group transmit processors instead of the transducer elements. Each intra-group receive processor 44ᵢ may include a summing delay line, or several programmable delay elements connected to a summing element (a summing junction). Each intra-group receive processor 44ᵢ delays the individual transducer signals, adds the delayed signals, and provides the summed signal to one channel 48ᵢ of receive beamformer 20. Alternatively, one intra-group receive processor provides the summed signal to several processing channels 48ᵢ of a parallel receive beamformer. The parallel receive beamformer is constructed to synthesize several receive beams simultaneously (multilines). Each intra-group receive processor 44ᵢ may also include several summing delay lines (or groups of programmable delay elements with each group connected to a summing junction) for receiving signals from several points simultaneously. A system controller 32 includes a microprocessor and an associated memory and is designed to control the operation of the ultrasound system. System controller 32 provides delay commands to the transmit beamformer channels via a bus 53 and also provides delay commands to the intra-group transmit processors via a bus 54. The delay data steers and focuses the generated transmit beams over transmit scan lines of a wedge-shaped transmit pattern, a parallelogram-shaped transmit pattern, or other patterns including three-dimensional transmit patterns. A system controller 32 also provides delay commands to the channels of the receive beamformer via a bus 55 and delay commands to the intra-group receive processors via a bus 56. The applied relative delays control the steering and focusing of the synthesized receive beams. Each receive beamformer channel 48ᵢ includes a variable gain amplifier which controls gain as a function of received signal depth, and a delay element that delays acoustic data to achieve beam steering and dynamic focusing of the synthesized beam. A summing element 50 receives the outputs from beamformer channels 48₁, 48₂, ... , , 48_{N} and adds the outputs to provide the resulting beamformer signal to an image generator 30. The beamformer signal represents a receive ultrasound beam synthesized along a receive scan line. Image generator 30 constructs an image of a region probed by a multiplicity of round-trip beams synthesized over a sector-shaped pattern, a parallelogram-shaped pattern or other patterns including three-dimensional patterns. Both the transmit and receive beamformers may be analog or digital beamformers as described, for example, in U.S. Pat. Nos. 4,140,022; 5,469,851; or 5, 345, 426.

The system controller controls the timing of the transducer elements by employing "coarse" delay values in transmit beamformer channels 41ᵢ and "fine" delay values in intra-group transmit processors 38ᵢ. There are several ways to generate the transmit pulses for the transducer elements. A pulse generator in the transmitter 10 may provide pulse delay signals to a shift register which provides several delay values to the transmit subarrays 30A. The transmit subarrays provide high voltage pulses for driving the transmit transducer elements. Alternatively, the pulse generator may provide pulse delay signals to a delay line connected to the transmit subarrays. The delay line provides delay values to the transmit subarrays, which provide high voltage pulses for driving the transmit transducer elements. In another embodiment the transmitter may provide shaped waveform signals to the transmit subarrays 30A. Further details concerning the transmit and receive circuitry of FIGURE 3 may be found in US Pat. 6,126,602.

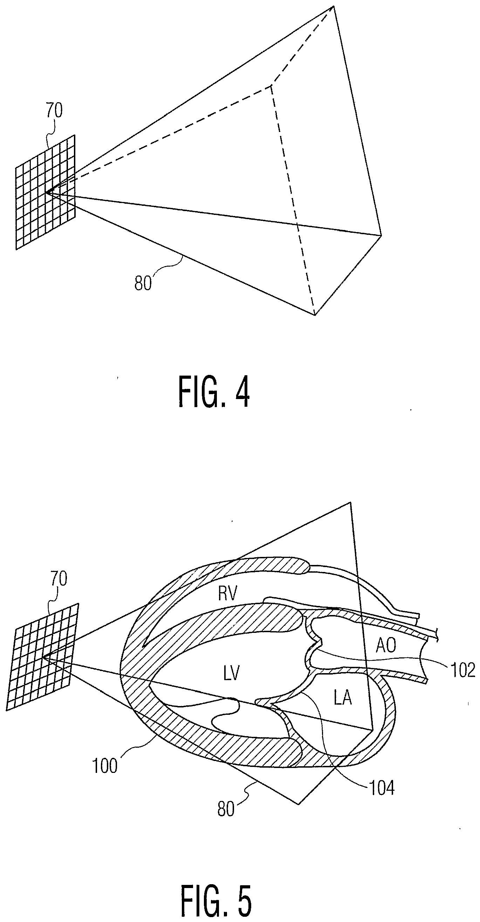

FIGURE 4 illustrates a two-dimensional matrix array transducer 70 which scans a volumetric region 80. By phased array operation of the transducer and imaging system described above, the matrix array can scan beams over a pyramidal volumetric region 80. The height of the pyramid from its apex to its base determines the depth of the region being imaged, which is chosen in accordance with factors such as the frequency and depth of penetration of the beams. The inclination of the sides of the pyramid are determined by the degree of steering applied to the beams, which in turn are chosen in consideration of the delays available for beam steering and the sensitivity of the transducer to off-axis (acutely inclined) beam steering.

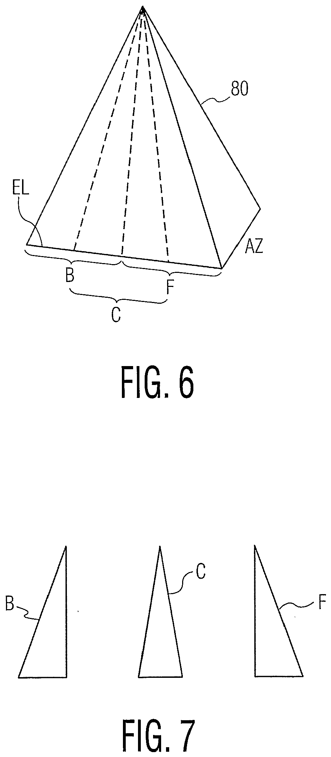

A maximal volumetric region such as volumetric region 80 may be of sufficient size to encompass the entire heart for 3D imaging as shown in FIGURE 5, in which the heart 100 is shown being apically scanned. Three chambers of the heart 100 are shown in the heart graphic of FIGURE 5, including the right ventricle (RA), the left atrium (LA), and the left ventricle (LV). Also shown is the aorta (AO) and its aortic valve 102, and the mitral valve 104 between the LA and the LV. However the time required to scan the entire maximal volumetric region 80 to visualize the entire heart may be too slow for satisfactory real time imaging, or may take too long such that motion artifacts occur, or both. In accordance with the principles of the present disclosure, the maximal volumetric region is divided into subvolumes B (back), C (center) and F (front), as shown in FIGURE 6. While the volumetric region 80 may subtend an angle in the azimuth (AZ) direction of 60°, for instance, the subvolumes will subtend lesser angles. In the embodiment of FIGURE 6 the subvolumes each subtend an angle of 30°. This means that, for the same beam density and depth, each subvolume can be scanned in half the time of the entire volumetric region 80. This will result in a doubling of the real time frame rate of display. The subvolumes can be made contiguous or overlapping. For example, if the angle of the maximal volumetric region were 90°, three contiguous subvolumes of 30° each might be employed. Alternatively, for a 60° maximal volumetric region, three 20° subvolumes could be used for an even higher frame rate. In the embodiment of FIGURE 6 the B and F subvolumes are contiguous in the center of the maximal volumetric region 80 and the C subvolume is centered at the center of the region 80. As explained below, this partitioning of the region 80 provides a constant, easy-to-comprehend reference of the 3D volumes for the benefit of the sonographer.

In accordance with a further aspect of the present disclosure each of the subvolumes is chosen by toggling a single control on the touchscreen 68 of the ultrasound system, enabling the sonographer to move through the sequence of subvolumes without moving the probe. In cardiac imaging, locating an acceptable acoustic window of the body is often challenging. Since the heart is enclosed by the ribs, which are not good conveyors of ultrasound, it is generally necessary to locate an aperture through the ribs or beneath the ribs for the probe. This is particularly difficult in 3D imaging, as the beams are steered in both elevation (EL) and azimuth. Once the sonographer finds an acceptable acoustic window to the heart, it is of considerable benefit to hold the probe in contact with the window during scanning. In an embodiment of the present disclosure the sonographer can locate the acoustic window while scanning the heart in 2D in the conventional manner. Once an acceptable acoustic window has been found during 2D imaging, the system is switched to 3D imaging with the touch of a button; there is no need to move the probe. The user can then step from the back to the center to the front subvolume with a single button, observing each subvolume in live 3D imaging and without the need to move the probe at any time.

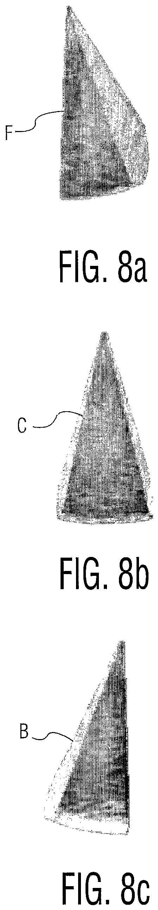

FIGURE 7 illustrates the profiles of each azimuthal center plane of each of the B, C, and F subvolumes formed as described above. When the three subvolumes are formed as illustrated in FIGURE 6, these center planes uniquely correspond to each subvolume: the center plane of the back subvolume B is a right triangle inclined to the left, the center plane of the front subvolume F is a right triangle inclined to the right and the center plane of the center subvolume C is symmetrical. As illustrated below, the shapes of these planes enable the sonographer to immediately comprehend the subvolume being viewed. FIGURES 8a, 8b, and 8c illustrate screen shots taken of a display screen 62 when the three subvolumes are displayed. In these and subsequent drawings the images have undergone black/white reversal from their conventional ultrasound display format for clarity of illustration. As just explained, the F subvolume in FIGURE 8a is seen to be inclined to the right, the B subvolume in FIGURE 8c is inclined to the left, and the C subvolume in FIGURE 8B is seen to be symmetrically balanced.

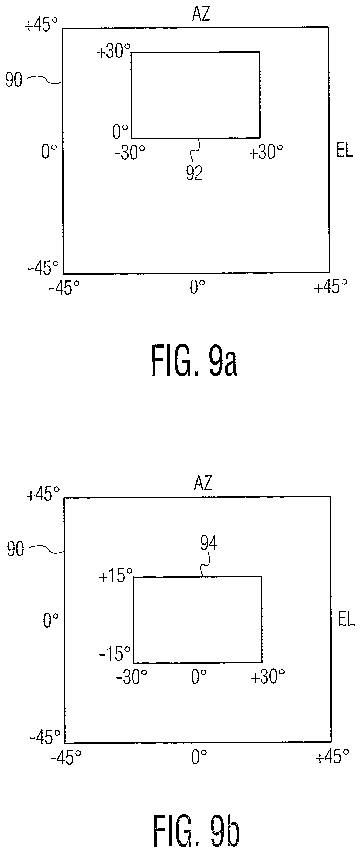

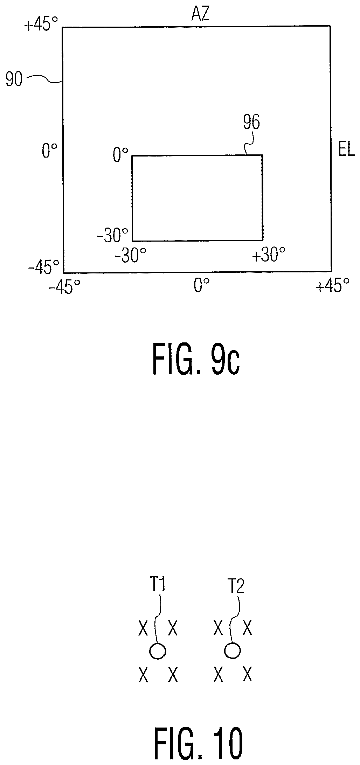

As a different subvolume is selected for viewing, the inclination of the beam planes of the transmit and receive beams is changed to acquire the desired subvolume. FIGURE 9a is a view normal to the plane of the matrix transducer which illustrates the beam scanning space in the theta-phi plane for 3D scanning. In this beam scanning space a row of beams in a horizontal line across the center of the aperture 90 extends normal to the face of the transducer in elevation but are steered progressively from left to right from -45° to 0° (in the center) to +45° in azimuth, as the transducer is operated as a phased array transducer. Similarly, a column of beams in a vertical line down the center of the aperture 90 extends normal to the face of the transducer in azimuth but are steered progressively from -45° to 0° (in the center) to +45° in elevation from the bottom to the top of the array. In FIGURE 9a a group of beam planes inclined from 0° to +30° is used to scan the front subvolume F. Each elevational beam plane extends from -30° to +30° in azimuthal inclination in this illustrated embodiment. When probe is stepped to scan the center subvolume C the transmit beam planes extend from a -15° inclination to a +15° inclination as shown in FIGURE 9b. When the probe is stepped to scan the back subvolume B the transmit beam planes used are those inclined from -30° to 0° as shown in FIGURE 9c. In each of these illustrations the beams in the beam plane are symmetrically inclined in azimuth from -30° to +30°. However in a constructed embodiment other inclinations could be used and/or the subvolume could be inclined asymmetrically to the left or right in azimuth as desired. Since the selection of the transmit and receive beam inclinations is done electronically by the system controller and the transmitter, there is again no need to move the probe from its acoustic window when making this change.

In a linear array embodiment, in which all of the beams are normal to the plane of the transducer, the transmit and receive apertures would be stepped along the array to transmit and receive spatially different subvolumes.

In a constructed embodiment 4X multiline is used to increase the beam density, which means that four receive beams are formed in response to each transmitted beam. FIGURE 10 shows a typical 4X multiline pattern, in which each transmit beam, T1 and T2 in this illustration, results in four receive beams represented by the four x's located around each transmit beam.

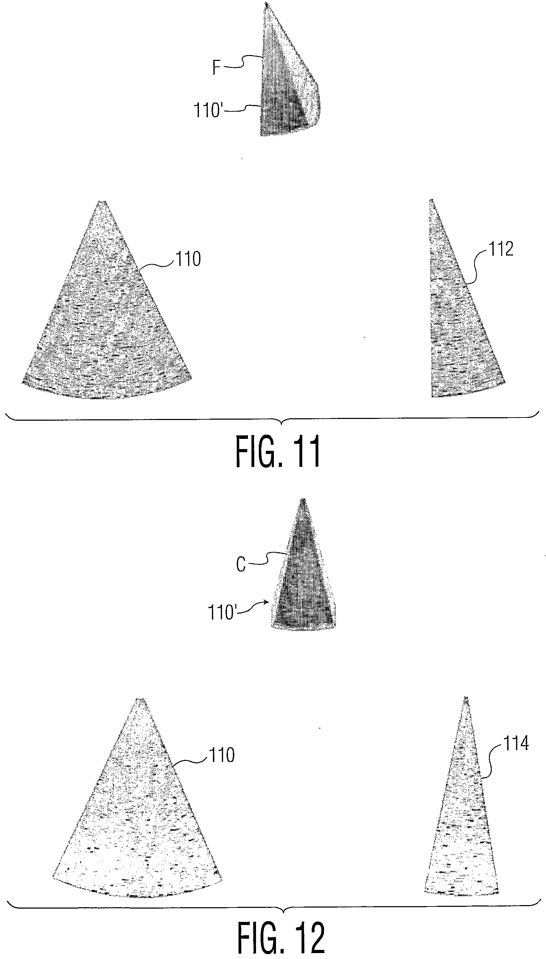

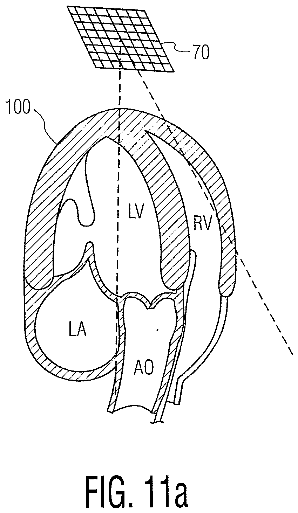

In accordance with another aspect of the present disclosure, each 3D subvolume display is also accompanied by two 2D images which help the sonographer orient the image being viewed. As previously explained, the sonographer begins by scanning the heart in 2D, moving the probe until an appropriate acoustic window is found. In this survey mode of operation, the matrix array probe is transmitting and receiving a single 2D image plane oriented normal to the center of the array. Once the acoustic window is found the 2D image is the center image plane of the maximal volumetric region 80 of FIGURE 6. The user then touches the "3D" button on the touchscreen 68 to switch to 3D imaging, and a single 3D image appears on the screen. The user can then touch the "Image" button on the touchscreen to see a number of display options. In a constructed embodiment one of these buttons has three triangles on it ("3Δ"), and when this button is touched the display screen 62 shows the three images shown in FIGURE 11, which is a B/W inverted actual screen shot. At the top center of the screen is the front subvolume F 3D image. At the lower left of the screen is a 2D image 110 of the face 110' of the subvolume F. When the three subvolumes are chosen as shown in FIGURE 6, the image 110 is also the center image of the maximal volumetric region 80 and is also the guiding 2D azimuthal image plane used in the initial 2D survey mode. On the lower right side of the display is a 2D image 112 of the center cut plane of the subvolume F, which is an elevation reference image in the illustrated embodiment. It is seen that the image 112 bears the distinctive profile of the front subvolume discussed in conjunction with FIGURE 7. Thus these orthogonal 2D images 110 and 112 provide familiar 2D assistance to the user in comprehending the orientation of the 3D subvolume image F. The subvolume F is the subvolume subtended by the dashed lines extending from the matrix array transducer 70 through the heart graphic 100 in FIGURE 11a.

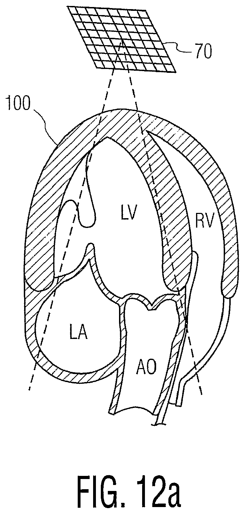

Also on the touchscreen 68 at this time is a button denoted "Front", for the F image view. When the user touches this button, it changes to a "Center" button and the display of FIGURE 12 appears on the display screen 62. The display has now switched to the 3D center subvolume C at the top of the screen. The 2D image 110 is an image of the center cut plane of this subvolume from the near side to the far side of the subvolume C as indicated by 110'. The symmetrical 2D image 114 is the distinctive symmetrical cut plane through the center of the subvolume from left to right. The subvolume C is that subtended by the dashed lines extending from the matrix transducer 70 in FIGURE 12a through the heart graphic 100.

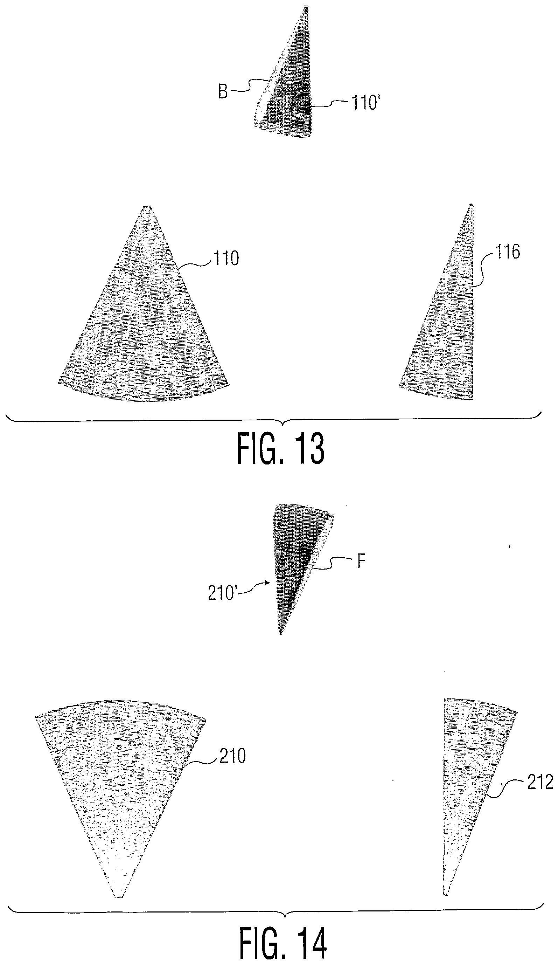

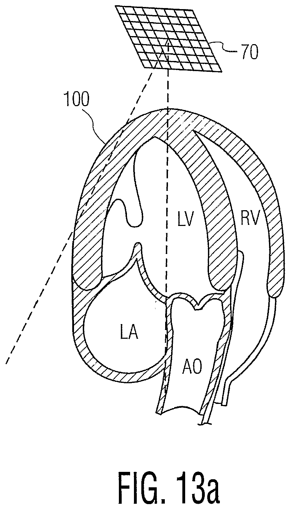

When the Center button is touched again it changes to read "Back" and the image display of FIGURE 13 appears with the 3D subvolume B shown at the top of the display. The 2D image 110 is still the center plane of the maximal volume in this embodiment (FIGURE 6), and is also the face plane on the right side 110' of the subvolume B. The distinctive center cut plane from left to right through the subvolume B is shown at 116. The volumetric subregion shown in this display is the region subtended by the dashed lines extending from the matrix transducer 70 in FIGURE 13a through the heart graphic 100.

Continual touching of the Front/Center/Back button will continue to switch the display through these three image displays. The sequence of the images may be selected by the system designer. For instance, in a constructed embodiment, the initial image display is of the Back subvolume and the selection switch toggles the display through the Back/Center/Front views in sequence. Thus, the sonographer can visualize the entire heart in live 3D by stepping through the three high frame rate subvolumes in succession.

In each of the image displays of FIGURES 11-13 the viewing perspective of the live 3D subvolume can be adjusted by the user. The images initially appear in the perspectives seen in the drawings but can then by changed by the user by rotating the trackball on the control panel 66. As the trackball is manipulated the 3D subvolumes appear to rotate in the display, enabling the user to view the anatomy in each subvolume from the front, back, sides, or other rotated viewing perspectives. This is accomplished by changing the dynamic parallax rendering look direction in response to movement of the trackball.

In accordance with a further aspect of the present disclosure, the 3D image orientation may be varied in accordance with the preferences of the user. For example, adult cardiologists usually prefer to visualize an apical view of the heart with the apex of the heart and the apex of the image both at the top of the screen as shown in the preceding FIGURES 11-13. In this orientation the heart is essentially seen in an upside down orientation. Pediatric cardiologists, on the other hand, will usually prefer to view both the apex of the heart and the apex of the image at the bottom of the screen, in which the heart is viewed in its right side up anatomical orientation. To enable each user to view the heart as he or she is accustomed, an embodiment of the present disclosure will have an Up/Down Invert button. In the embodiment described below the ultrasound system also has a Left/Right Reversal button which is also described.

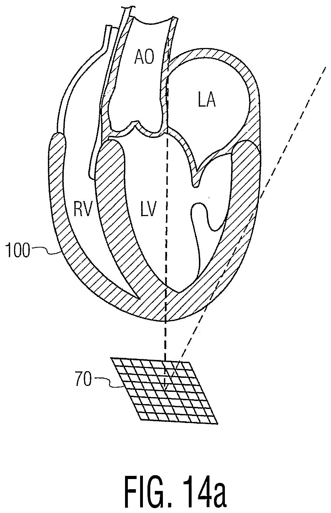

When the user touches the Up/Down Invert button on the touchscreen 68, the order in which the scanlines are processed for display in scan conversion and 3D rendering is reversed and the display will switch to that shown in FIGURE 14. In this view the 3D subvolume F has become inverted with the apex of the heart at the bottom of the image as illustrated by the matrix array 70 and the heart graphic 100 in FIGURE 14a. The center plane 210 of the maximal volumetric region 80 has also been correspondingly inverted and still illustrates the view of the face 210' of the inverted subvolume F. Likewise, the distinctive center cut plane 212 of the subvolume F is also inverted. Inversion of the image also reverses the left-right direction of the images on the display screen so that the original sense of the anatomy is retained in the images. In the illustrated embodiment inversion (and reversal, as discussed below) will cause the "Back" subvolume to become the "Front" subvolume, and *vice versa.*

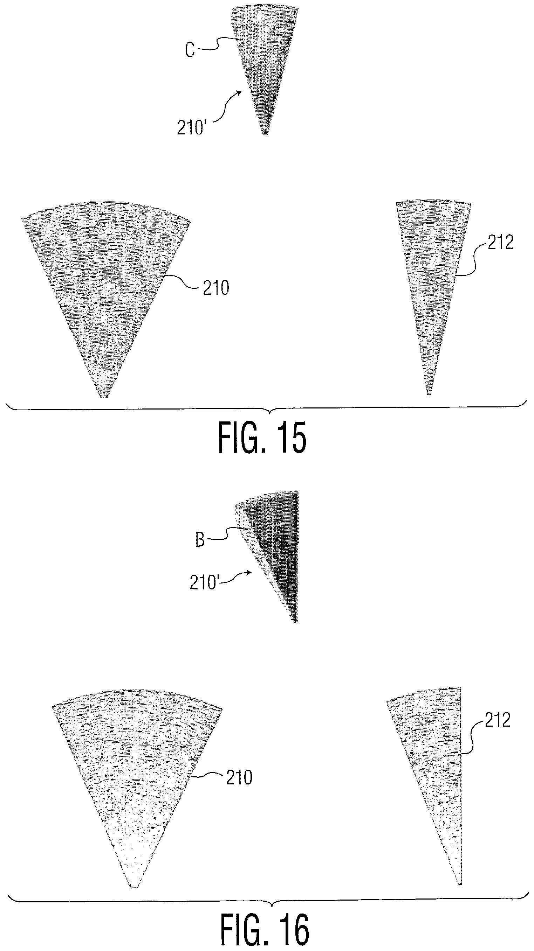

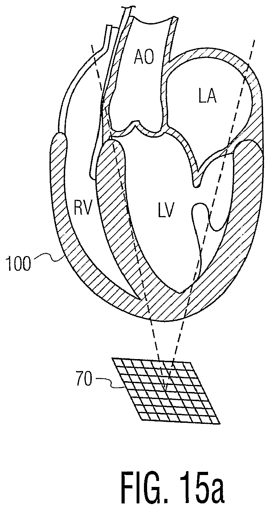

Touching the touchscreen button now reading Front will cause the button to change to Center and the display to switch to the inverted 3D center subvolume C as shown in FIGURE 15. The 2D front-to-back center plane 210 of the subvolume C is inverted, as is the distinctive left-to-right cut plane 212. The subvolume C is that acquired between the dashed lines extending from the matrix array transducer 70 through the heart graphic 100 in FIGURE 15a.

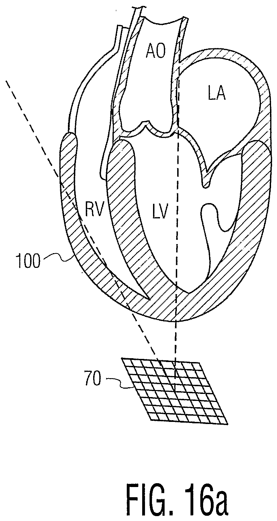

Touching the touchscreen button again will cause the button to change to Back and the display to change to that shown in FIGURE 16. The inverted 3D subvolume B is that acquired as illustrated by the dashed lines extending from the matrix transducer 70 through the heart graphic 100 in FIGURE 16a. The 2D center plane 210 is the side face 210' of the inverted subvolume in this embodiment, and the distinctive cut plane 212 of the subvolume B is also inverted.

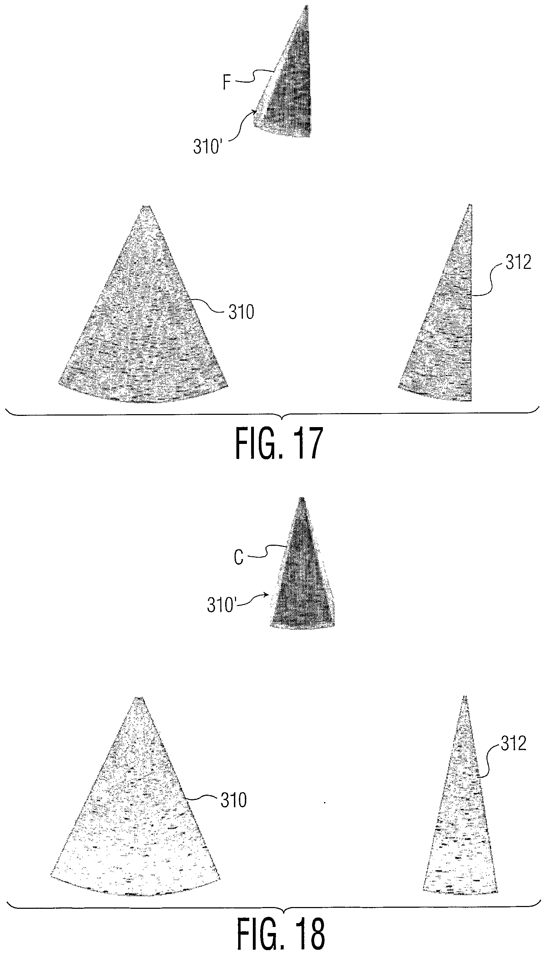

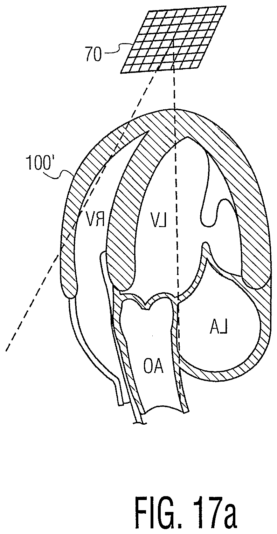

In accordance with a further aspect of the present disclosure, the left-right direction of the 3D images can also be reversed. When the Left/Right Reversal button on the touchscreen 68 is touched, the order of the scanlines used in the scan conversion and rendering display processes is reversed, causing the images to change sense from left to right. This effectively causes front to become back, and *vice versa* for the 3D subvolumes. For instance, FIGURE 17 shows a 3D subvolume F after left/right reversal. The subvolume is viewed as if the direction of the anatomy has been reversed as illustrated by the reverse image 100' of the heart in FIGURE 17a. The center plane 210 and the distinctive cut plane 312 in FIGURE 17 are correspondingly reversed in display line sequence.

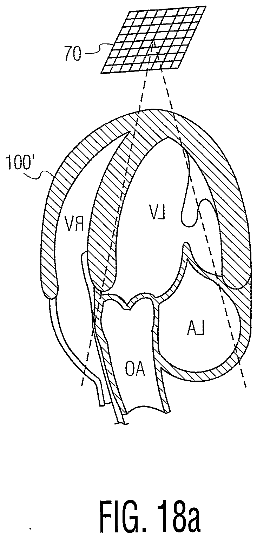

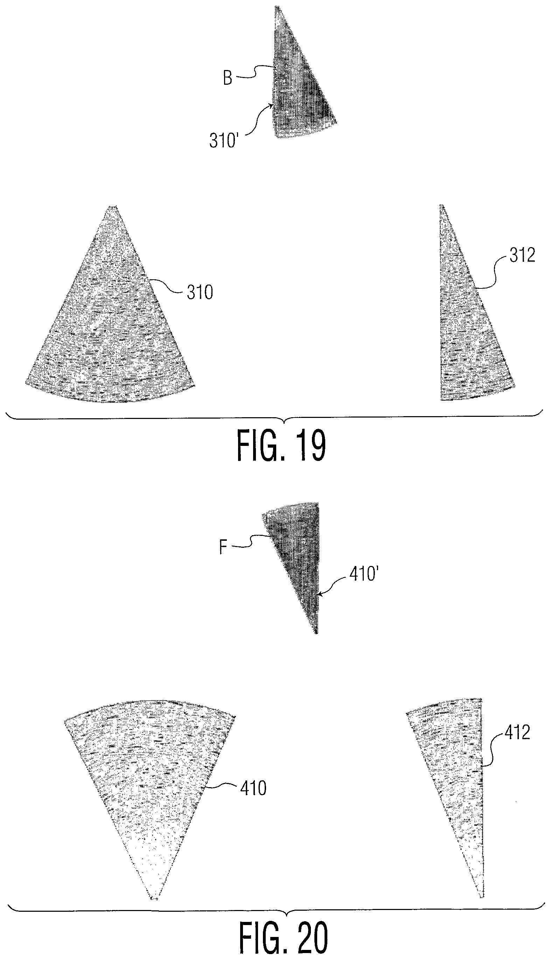

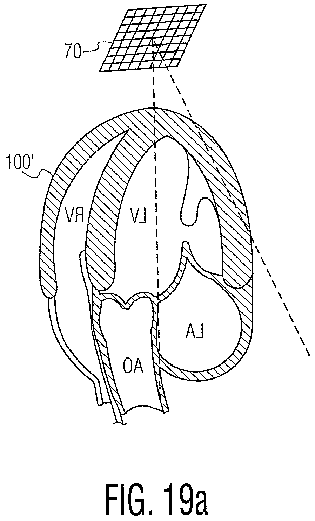

Sequencing through the Front/Center/Back button sequence will next cause a reversed 3D subvolume C image to appear as shown in FIGURE 18, as well as reversed center plane image 310 and left to right cut plane 312. The image reversal is indicated by the reversed heart graphic 100' in FIGURE 18a. When the touchscreen button is touched a third time a reversed 3D back subvolume image B appears as shown in FIGURE 19, together with reversed center plane image 310 and back cut plane image 312. The images are oriented as though the heart were reversed as shown in FIGURE 19a.

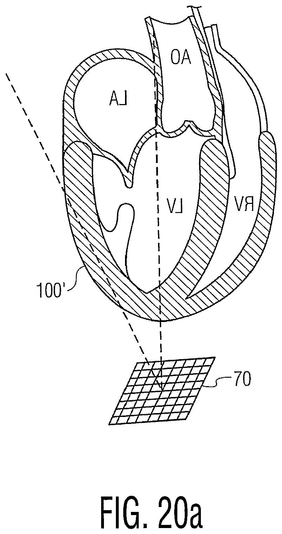

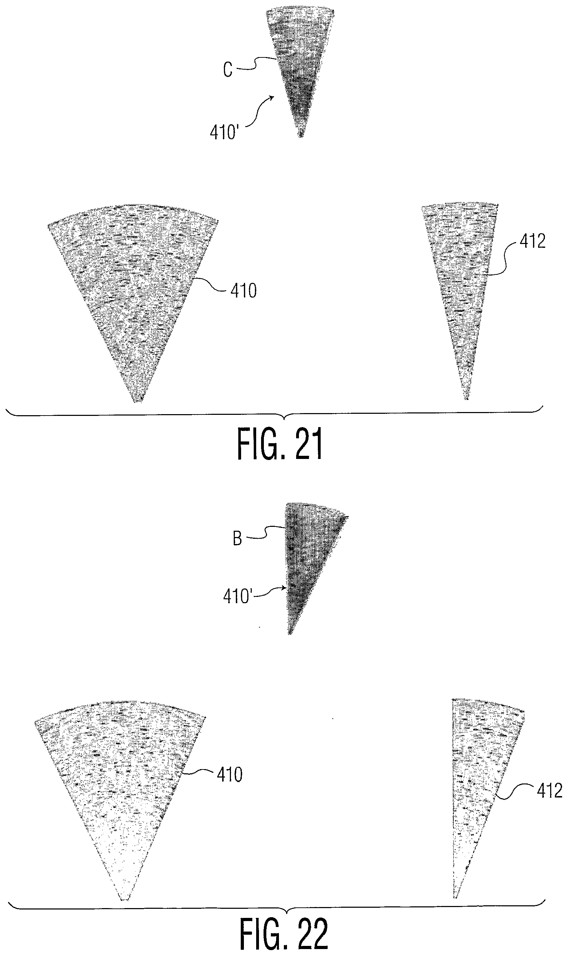

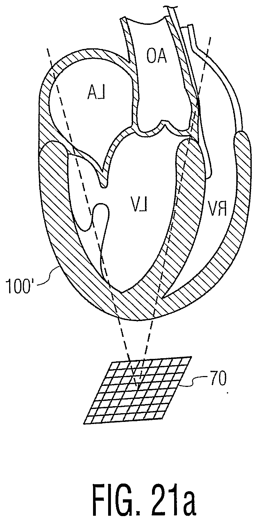

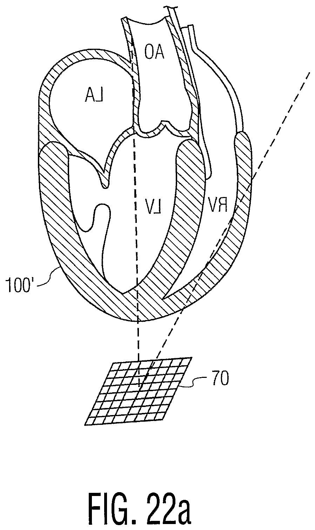

Finally, the Up/Down Inverted images can also be Left/Right Reversed as shown in FIGURES 20, 21, and 22 for the front, center and back subvolumes. In this sequence the heart appears as if both inverted and reversed as shown by the heart graphic 100' in FIGURE 20a, 21a, and 22a. With both up/down inversion and left/right reversal the object being scanned can be viewed from any orientation, as if the user were scanning the anatomy from different perspectives of the body.

The aforedescribed embodiments effectively step the sonographer through incrementally positioned subvolumes of the maximal volumetric region. Rather than step through a series of discretely positioned orientations, it may be desirable to continuously change the orientation of a subvolume. This is done by touching the "Volume Steer" button on the touchscreen 68 when the user is in the 3D mode. In the volume steer mode the user can manipulate a continuous control on the control panel 66 such as a knob or trackball to sweep the displayed volume back and forth. In a constructed embodiment one of the knobs below the touchscreen 68 is used as the volume steer control, and a label on the touchscreen above the knob identifies the knob as the volume steer control. When the system enters the volume steer mode, the 3D subvolume shown on the screen can be reoriented with the control knob. When the volume steer knob is turned to the right the displayed subvolume appears to swing to the right from its apex, and when the knob is turned to the left the displayed subvolume appears to swing to the left. A subvolume can be steered in this manner in inverted, uninverted, reversed or unreversed viewing perspective. The motion appears continuous, corresponding to the continuous motion of the knob.

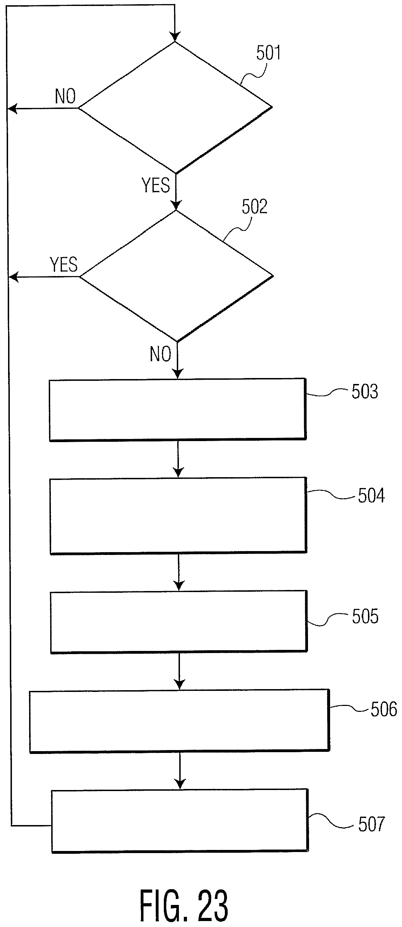

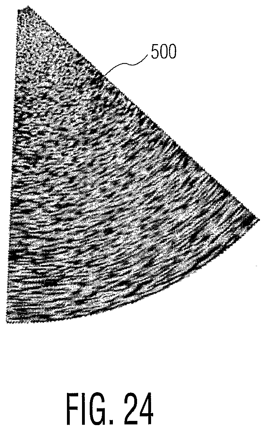

The control sequence for this continuous mode of volume steering is shown in the flowchart of FIGURE 23. While the system is in this mode the system controller is continually monitoring any change in the volume steer knob. If no movement is sensed, this monitoring continues as shown in step 501. If a change in the knob position is sensed ("Yes"), the controller checks in step 502 to see if the subvolume is at a limit of the maximal volumetric region over which volume steering is permitted (*e.g.,* in contact with a side of maximal volume 80.) If the subvolume has been steered to its limit, the system goes back to monitoring for a change in knob position, as only a knob change in the other direction will swing the subvolume. If a limit position has not been reached, the beam steering angles for the transmit and receive beamformers are incremented in accordance with the change in knob position to steer the volume in the slightly different orientation in step 503. This volume geometry change is communicated to the scan converter of the display system in step 503 so that the newly acquired volumetric images will be shown in their new orientation. The beamformer controller computes the first beam position of the new volumetric orientation and the stop and start orientations of the beams in step 504. The parameters for scan conversion to the new orientation are reset in step 505. The new beam parameters for the transmit and receive beamformers are set in step 506. The system then commences to acquire and display the 3D subvolume in its new orientation such as that shown in the screen shot of FIGURE 24, and the system controller resumes monitoring of the volume steer control knob for a subsequent change. With this mode of operation the sonographer can electronically sweep a 3D subvolume back and forth over the range limits of the maximal volumetric region to acquire high frame rate 3D images within the maximal volumetric region without the need to move the probe from its acoustic window. In a constructed embodiment subvolumes subtending angles as great as 57° have been swept over a maximal volumetric region subtending as much as 90°.

## Claims

1. An ultrasonic diagnostic imaging system for three dimensional imaging comprising:
a matrix array transducer which is operable to scan electronically steerable beams over a volumetric region of a body and receive scanlines in response to the beam scanning;
a volume rendering image processor coupled to the matrix array transducer which processes received scanlines in a given order to produce live 3D images of the volumetric region in a given orientation;
a display coupled to the image processor which displays the live 3D images; and
a user control coupled to the volume rendering image processor,
**characterized in that** the
the user control is adapted to cause the received scanlines to be processed in a reversal of the given order such that the live 3D images on the display appear in an inverted orientation.

2. The ultrasonic diagnostic imaging system of Claim 1, wherein the volumetric region comprises anatomy having a top, a bottom, a left side, and a right side when viewed from a given viewing perspective; wherein the top of the anatomy as seen on the display prior to inversion appears at the bottom of the image after inversion, the bottom of the anatomy as seen on the display prior to inversion appears at the top of the image after inversion, the left side of the anatomy as seen on the display prior to inversion appears at the right side of the image after inversion, and the right side of the anatomy as seen on the display prior to inversion appears at the left side of the image after inversion.

3. The ultrasonic diagnostic imaging system of Claim 1, wherein the display displays the 3D image in a sector format.

4. The ultrasonic diagnostic imaging system of Claim 1, further comprising:
a scan converter coupled to the matrix array transducer and responsive to the user control which processes received scanlines in a given order to produce a 2D image of a plane of the volumetric region in a given orientation;
wherein the display is coupled to the scan converter for display of a 2D image; and
wherein the user control is further adapted to cause the scan converter to process received scanlines in a reversal of the given order and the 2D image on the display to appear in an inverted orientation.

5. The ultrasonic diagnostic imaging system of Claim 4,
wherein the user control is operable to simultaneously invert both the live 3D image and the 2D image on the display.

6. A method for changing the orientation of a live 3D ultrasound image comprising:
providing a matrix array transducer, which is operable to scan electronically steerable beams over a volumetric region of a body and receive scanlines in response to the beam scanning;
providing a volume rendering image processor coupled to the matrix array transducer, which processes received scanlines in a given order to produce live 3D images of the volumetric region in a given orientation;
displaying the live 3D image in the given orientation on a display;
providing a user control coupled to the volume rendering image processor; and
**characterized in that** the method further comprises
actuating the user control, which causes the received scanlines to be processed in a reversal of the given order such that the live 3D images on the display appear in an inverted orientation.

## Patentansprüche

1. Diagnostisches Ultraschallbildgebungssystem für die dreidimensionale Bildgebung, das Folgendes umfasst:
einen Matrixarraywandler, der betriebsfähig ist, um eine volumetrische Region eines Körpers mit elektronisch lenkbaren Strahlenbündeln abzutasten und in Reaktion auf das Abtasten mit dem Strahlenbündel Abtastlinien zu empfangen;
einen mit dem Matrixarraywandler gekoppelten Volumenrendering-Bildprozessor, der die empfangenen Abtastlinien in einer gegebenen Reihenfolge verarbeitet, um Live-3D-Bilder der volumetrischen Region in einer gegebenen Ausrichtung zu erzeugen;
eine mit dem Bildprozessor gekoppelte Anzeige, die die Live-3D-Bilder anzeigt; und
eine mit dem Volumenrendering-Bildprozessor gekoppelte Benutzersteuerung,
**dadurch gekennzeichnet, dass** die Benutzersteuerung dafür ausgelegt ist, zu veranlassen, dass die empfangenen Abtastlinien in einer Umkehrung der gegebenen Reihenfolge verarbeitet werden, so dass die Live-3D-Bilder auf der Anzeige in einer invertierten Ausrichtung erscheinen.

2. Diagnostisches Ultraschallbildgebungssystem nach Anspruch 1, wobei die volumetrische Region aus einer gegebenen Blickrichtung gesehen Anatomie mit einer oberen, einer unteren, einer linken und einer rechten Seite umfasst; wobei die obere Seite der Anatomie, wie sie vor der Invertierung auf der Anzeige dargestellt wird, nach der Invertierung im unteren Bereich des Bilds erscheint, die untere Seite der Anatomie, wie sie vor der Invertierung auf der Anzeige dargestellt wird, nach der Invertierung im oberen Bereich des Bilds erscheint, die linke Seite der Anatomie, wie sie vor der Invertierung auf der Anzeige dargestellt wird, nach der Invertierung auf der rechten Seite des Bilds erscheint, und die rechte Seite der Anatomie, wie sie vor der Invertierung auf der Anzeige dargestellt wird, nach der Invertierung auf der linken Seite des Bilds erscheint.

3. Diagnostisches Ultraschallbildgebungssystem nach Anspruch 1, wobei die Anzeige das 3D-Bild in einem Sektorformat anzeigt.

4. Diagnostisches Ultraschallbildgebungssystem nach Anspruch 1, das weiterhin Folgendes umfasst:
einen mit dem Matrixarraywandler gekoppelten und auf die Benutzersteuerung reagierenden Signalwandler, der die empfangenen Abtastlinien in einer gegebenen Reihenfolge verarbeitet, um ein 2D-Bild einer Ebene der volumetrischen Region in einer gegebenen Ausrichtung zu erzeugen;
wobei die Anzeige mit dem Signalwandler gekoppelt ist, um ein 2D-Bild anzuzeigen; und
wobei die Benutzersteuerung weiterhin dafür ausgelegt ist, den Signalwandler zu veranlassen, die empfangenen Abtastlinien in einer Umkehrung der gegebenen Reihenfolge zu verarbeiten und das 2D-Bild in einer invertierten Ausrichtung auf der Anzeige erscheinen zu lassen.

5. Diagnostisches Ultraschallbildgebungssystem nach Anspruch 4,
wobei die Benutzersteuerung betriebsfähig ist, um gleichzeitig sowohl das Live-3D-Bild als auch das 2D-Bild auf der Anzeige zu invertieren.

6. Verfahren zum Ändern der Ausrichtung eines Live-3D-Ultraschallbilds, umfassend:
Bereitstellen eines Matrixarraywandlers, der betriebsfähig ist, um eine volumetrische Region eines Körpers mit elektronisch lenkbaren Strahlenbündeln abzutasten und in Reaktion auf das Abtasten mit dem Strahlenbündel Abtastlinien zu empfangen;
Bereitstellen eines mit dem Matrixarraywandler gekoppelten Volumenrendering-Bildprozessor, der die empfangenen Abtastlinien in einer gegebenen Reihenfolge verarbeitet, um Live-3D-Bilder der volumetrischen Region in einer gegebenen Ausrichtung zu erzeugen;
Anzeigen der Live-3D-Bilder in einer gegebenen Ausrichtung auf der Anzeige;
Bereitstellen einer mit dem Volumenrendering-Bildprozessor gekoppelten Benutzersteuerung; und
**dadurch gekennzeichnet, dass** das Verfahren weiterhin umfasst:
Betätigen der Benutzersteuerung, wodurch veranlasst wird, dass die empfangenen Abtastlinien in einer Umkehrung der gegebenen Reihenfolge verarbeitet werden, so dass die Live-3D-Bilder auf der Anzeige in einer invertierten Ausrichtung erscheinen.

## Revendications

1. Système d'imagerie diagnostique à ultrasons pour imagerie en trois dimensions comprenant :
un transducteur à réseau matriciel qui sert à balayer des faisceaux électroniquement orientables sur une région volumétrique d'un corps et à recevoir des lignes de balayage en réponse au balayage de faisceau ;
un processeur d'image à rendu de volume couplé au transducteur à réseau matriciel qui traite des lignes de balayage reçues dans un ordre donné pour produire des images en 3D en direct de la région volumétrique dans une orientation donnée ;
un dispositif d'affichage couplé au processeur d'image qui affiche les images en 3 D en direct ; et
une commande utilisateur couplée au processeur d'image à rendu de volume,
**caractérisé en ce que**
la commande utilisateur est adaptée pour amener les lignes de balayage reçues à être traitées lors d'un renversement de l'ordre donné de telle sorte que les images en 3D en direct sur le dispositif d'affichage apparaissent dans une orientation inversée.

2. Système d'imagerie diagnostique à ultrasons selon la revendication 1, dans lequel la région volumétrique comprend une anatomie ayant une partie supérieure, une partie inférieure, un côté gauche et un côté droit quand on l'observe depuis une perspective de visualisation donnée ; dans lequel la partie supérieure de l'anatomie telle que vue sur le dispositif d'affichage avant inversion apparaît au niveau de la partie inférieure de l'image après inversion, la partie inférieure de l'anatomie telle que vue sur le dispositif d'affichage avant inversion apparaît au niveau de la partie supérieure de l'image après inversion, le côté gauche de l'anatomie tel que vu sur le dispositif d'affichage avant inversion apparaît au niveau du côté droit de l'image après inversion, et le côté droit de l'anatomie tel que vu sur le dispositif d'affichage avant inversion apparaît au niveau du côté gauche de l'image après inversion.

3. Système d'imagerie diagnostique à ultrasons selon la revendication 1, dans lequel le dispositif d'affichage affiche l'image en 3D selon un format de secteur.

4. Système d'imagerie diagnostique à ultrasons selon la revendication 1, comprenant en outre :
un convertisseur de balayage couplé au transducteur à réseau matriciel et répondant à la commande utilisateur qui traite des lignes de balayage reçues dans un ordre donné pour produire une image en 2D d'un plan de la région volumétrique dans une orientation donnée ;
dans lequel le dispositif d'affichage est couplé au convertisseur de balayage pour affichage d'une image en 2D ; et
dans lequel la commande utilisateur est en outre adaptée pour amener le convertisseur de balayage à traiter les lignes de balayage reçues lors d'un renversement de l'ordre donné et l'image en 2D sur le dispositif d'affichage à apparaître dans une orientation inversée.

5. Système d'imagerie diagnostique à ultrasons selon la revendication 4,
dans lequel la commande utilisateur sert à inverser simultanément l'image en 3D en direct et l'image en 2D sur le dispositif d'affichage.

6. Procédé pour changer l'orientation d'une image à ultrasons en 3D en direct comprenant :
la fourniture d'un transducteur à réseau matriciel, qui sert à balayer des faisceaux électroniquement orientables sur une région volumétrique d'un corps et à recevoir des lignes de balayage en réponse au balayage de faisceau ;
la fourniture d'un processeur d'image à rendu de volume couplé au transducteur à réseau matriciel, qui traite des lignes de balayage reçues dans un ordre donné pour produire des images en 3D en direct de la région volumétrique dans une orientation donnée ;
l'affichage de l'image en 3D en direct dans l'orientation donnée sur un dispositif d'affichage ;
la fourniture d'une commande utilisateur couplée au processeur d'image à rendu de volume ; et
**caractérisé en ce que** le procédé comprend en outre
l'actionnement de la commande utilisateur, qui amène les lignes de balayage reçues à être traitées lors d'un renversement de l'ordre donné de telle sorte que les images en 3D en direct sur le dispositif d'affichage apparaissent dans une orientation inversée.
